# EUROPEAN PATENT APPLICATION

(11) **EP 0 754 455 A1**
(43) Date of publication of application: **22.01.1997**
(21) Application number: 96901807.6
(22) Date of filing: 08.02.1996
(51) Int. Cl.: A61K 31/40, C07D 209/14

(54) **PHARMACEUTICAL COMPOSITIONS BASED ON AMINOMETHYLINDOLES AND THEIR THERAPEUTICAL APPLICATION AS NEUROPROTECTORS IN PARKINSON AND ALZHEIMER DISEASES**

(30) Priority: 09.02.1995 ES 9500271
(71) Applicant: CONSEJO SUPERIOR INVESTIGACIONES CIENTIFICAS (CSIC), 28006 Madrid (ES); Universidad Autonoma Barcelona, 08193 Bellaterra (ES)
(72) Inventor: FERNANDEZ ALVAREZ, Eldiberto Inst.Quimica Org.Gen., Juan de la Cierva, 3 28006 Madrid (ES); UNZETA LOPEZ, Mercedes, E-08193 Bellaterra (ES); MARCO CONTELLES, José Luis Inst. Quim. Orgán. Gen., Juan de la Cierva, 3 28006 Madrid (ES)
(74) Representative: Curell Sunol, Jorge
(86) International application number: ES9600025
(87) International publication number: WO9624349

(57) **Abstract**

Pharmaceutical compositions containing as active components 2-aminomethyl-5-benzyloxyindoles derivatives, including the N-propinyl, N-2-butinyl and N-2,3-butadinylderivatives which are potent inhibitors of monoaminoxydase A and B of rat liver and brain, as well as of the human brain, with marked selectivity for the in vitro and in vivo inhibition of monoaminoxydase B, preferably on the A. In relation to said selectivity of inhibition of monoaminoxydase B, as well as to their capacity to neutralize in laboratory animals the neurotoxic action of MPTP and other neurotoxines, the disclosed compounds are useful for the treatment of cerebral neurodegenerative processes, and particularly for the treatment of Parkinson and Alzheimer diseases.

## Description

The present invention refers to 2-aminomethyl-5-benzyloxyindols (formula I) and their use as therapeutic agents in pharmaceutical formulations that incorporate them as ingredients, to be used as neuroprotector agents in the neurodegenerative diseases of Parkinson and Alzheimer. Compounds of this type are very potent monoamine oxidase (MAO) inhibitors and highly selective for the MAO-B form.

In addition, these compounds are substrates of the cerebral aminergic re-uptake system and are therefore useful "in vivo" for altering the neurotransmitter levels at a CNS level. 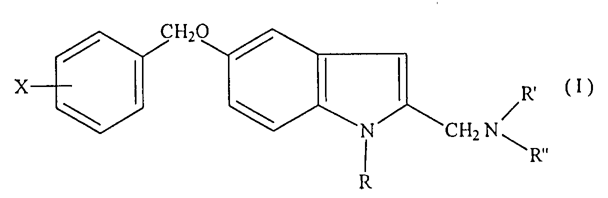
X = H, halogen, alkoxy, alkyl, alkylthio, aryl, aryloxi.
R = H, CH₃ or other aliphatic, alicyclic or aryl radicals.
R'= ^{H}, CH₃ or other aliphatic or alicyclic C₁ C₃ radicals, or an aryl or arylalkyl radical, or a radical the same as those indicated for R''.
R''= H, CH₃ or other aliphatic or alicyclic C₁ C₃ radical, or an acetylene or alene group, especially those represented by propinyl, 2-butinyl or 2-3-butadienyl radicals.

In the central nervous system (CNS) the activity of monoamine oxidase (EC1.4.3.4) (MAO) metabolises biogenic amines with a neurotransmitter function. At a physiological level, it is the enzyme responsible for maintaining the monoaminergic tone. MAO appears in two different molecular forms: MAO-A, that preferably deaminates serotonin (5-HT) and is which selectively inhibited by chlorgilin and MAO-B whose substrate is preferably phenylethylamine (PEA) and is specifically inhibited by deprenyl. In the brain tissue. both enzymatic forms have a different distribution, thus paradoxically MAO-A appears in dopaminergic and noradrenergic neurons, whereas MAO-B is localised in serotonergic neurons and glial cells.

In addition to its role in the metabolism of amine neurotransmitters, other regulatory functions have also been attributed to MAO-B. MAO-B has been described as having a key role in maintaining the concentration of certain trace amines such as PEA, which modulates dopaminergic transmission in the CNS. (Paterson et al. 1990, J. Neurochem 55:1827-1837). On the other hand, MAO-B has been observed as implicated in the metabolism of certain exogenic compounds including 1-methyl-4-phenyl-1,2,3,6 tetrahydropyridine (MPTP) protoxin which is metabolised by MAO-B to produce the 1-methyl-4-phenylpyridine ion (MPP) the true neurotoxin that develops the same symptoms in primates as Parkinson's disease. (Heikkila RE et al. 1990, J Neural Trans, 32:217-227).

On the other hand, in various processes that involve neuronal death such as cerebral ischemia or certain neurodegenerative diseases, activity of the dopaminergic metabolism has been observed together with an increase in the MAO-B activity. It has been suggested that the resulting excessive generation of H₂O₂ by the metabolic action of this enzyme leads to oxidative stress, an increase of the lipidic perioxidation and the consequent alteration of neuronal membrane functionality (Cohen et al., 1990, J Neural Transm, [Suppl] 32:229-238. This situation also occurs in patients with Parkinson's disease, where a large part of the dopaminergic nigra-striatal system has degenerated, with the corresponding appearance of an increase in dopamine turnover. It has also been noted that the cerebral activity of MAO increases with age, especially that of MAO-B. This increase has been attributed to the gliosis associated with aging (Fowler CJ, J Neural Transm. 1990 49:1-20). On the other hand, in Alzheimer's disease, characterised by serious cognitive and neuropathological alterations, increases of MAO-B activity have also been recorded, (Jossan SS et al. J Neural Transm, 1990 [Suppl 32]:61-65), perhaps due to a new form of MAO-B (Steventon et al. 1990, The Lancet, Vol 3 (86-82):180). Later MAO-B inhibitors (MAO-BI) were described for therapy in this disease (Mangoni et al. 1991, Eur Neurol. 31:100-107).

Given the implication of MAO-B in the above neurological dysfunctions, considerable effort has been made to obtain potent, selective, specific inhibitors that would permit control over this enzymatic activity. Proof of this is the existence of the DATATOP multicentre group (Deprenyl and Tocopherol Antioxidative Therapy of Parkinsonism) that promotes the use of MAO-BIs, especially deprenyl, as preferential therapy in the treatment of Parkinsonism. In addition, they have also been described as useful in the treatment of certain depressive subtypes, Alzheimer's disease and to retard the effects associated with aging (Knoll J, 1982, Keverling Buisman JA Eds. Strategy in Drug Research, Elsevier Amsterdam, pp 107-135). In spite of the therapeutic efficacy of deprenyl, its use in the treatment of Parkinsonism produces little symptomatic improvement in the elderly and only allows retarding the disease in younger patients. On the other hand, its use is not free of side effects that advise against prolonged use (Wessel K, 1993, Inhibitors of Monoamine Oxidase B, Szelenyi Eds, Cap. 13).

As a result of the above, there is currently an increasing interest in the development of new pharmaceutical products and compositions for the treatment of neurodegenerative processes. Among these, Parkinson's and Alzheimer's disease are of special interest due to the increase in life expectancy. They affect 10% of the population older than 65 and more than 40% of those over 80 years of age.

At present, in spite of the very large number of MAO-A inhibitors that are known and used in antidepression therapy, there are relatively few selective MAO-B inhibitors available on the market. It is also important to mention that the structural reasons determining their greater potency and selectivity are still unknown.

In a series of Spanish patents (No. 8900727/1989, 8900452/1989, 8900562/1989) and one European patent (No. 90301346/1990), we described the method for the synthesis of a wide range of 2-aminomethylindole derivatives with different substitutions based on their possible interest as inhibitors of monoamine oxidases A and B. In particular, Spanish patent No. 8900727/1989 describes the preparation of 2-aminomethyl-5-benzyloxyindoles and its general formula N-acetylene and N-alene derivatives (I), as well as the preliminary results of their biological assessment as inhibitors of monoamine oxidase A and B, using the enzyme from mitochondrias of bovine brain. 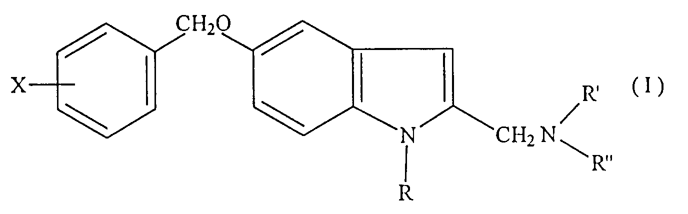
X = H, halogen, alkoxy, alkyl, alkyltio, aryl, aryloxy.
R = H, CH₃ or other aliphatic. alicyclic or aryl radicals.
R'= H, CH₃ or other aliphatic or alicyclic C₁ C₃ radicals, or an aryl or arylalkyl, or a radical the same as those indicated for R''.
R''= H, CH₃, or other aliphatic or alicyclic C₁-C₃ radical, or an acetylene or alene group, especially those represented by propinyl, 2-butinyl or 2,3-butadienyl radicals.

The results of this biological study (MA Cruces, E Elorriaga, E Fernández Alvarez, Eur. J. Med. Chem. 26, 33-41, 1991; Biochem Pharmacol 40, 535-543, 1990), using bovine brain as tissue and a substrate not specific for either of the two forms of monoamine oxidase A and B (tyramine), showed that compounds with the general formula (I), and especially the derivatives with acetylene or alene groups were very potent irreversible monoamine oxidase inhibitors, but that they did not show any selectivity.

The preliminary data obtained under the above conditions indicated that, because of their lack of selectivity, the tested compounds were not candidates for pharmacological or therapeutic use and further study as discontinued.

These results, based only on the respective values for IC₅0, were used in a later study of the structural-activity relationship (C. Cativiela et al. Acta Chim Hungar - Mode in Chem 130 (1) 129-143, 1992), which, logically, did not allow any definite conclusion to be drawn.

At present, using more sophisticated test methods (Avila M et al 1993 Biochem Pharmacol 45, 2231-2237) and human and rat tissue to obtain the isolated forms of monoamine oxidase A and B, as well as specific substrates for each (¹4C-serotonin and ¹4C-2-phenylethylamine, MAO-A and MAO-B respectively) we have studied the family of compounds derived from the general formula (I).

In vitro studies of experimental animals, as well as those carried out in human brains post-mortem and the pertinent pharmacological tests, indicate the high potency and selectivity of these compunds as monoamine oxidase B inhibitors, as well as their possible therapeutic application as neuroprotectors in Parkinson's and Alzheimer's disease.

### RESULTS OF IN VITRO STUDIES ON EXPERIMENTAL ANIMALS

### Kinetic inhibition studies

The following system has been used for the biological assessment of the 5-benzyloxy derivatives used in the previous studies:
- Determination of the IC₅0 compared to specific substrates of MAO-A (serotonin) and MAO-B (phenylethylamine) at time 0 and 30 minutes of incubation.
- Confirmation of the time dependence of the inhibition process.
- Demonstration of the irreversibility of the inhibition process.
   Once these stages had been completed, a suicide type, highly selective inhibition behaviour was confirmed.
- Determination of the kinetic parameters using continuous spectrophotometric techniques.

### RESULTS OF IN VITRO STUDIES ON HUMANS

The biological assessment of the 5-benzyloxy derivatives in post-mortem human brain tissue was carried out according to the following system:
- Determination of the IC₅0 compared to specific substrates of MAO-A (serotonin) and MAO-B (phenylethylamine) at time 0 and 30 minutes of incubation.
- Determination of the IC₅0 of the dopamine carrier.

### Example 1. Determination of the IC₅0 in experimental animals.

The mitochondrial portion of Sprague-Dawley rat was used to determine the MAO-A and MAO-B activity. As both enzymes are found in a proportion of 1:1 in this tissue, it was necessary to obtain each activity separately, by selective inhibition with chlorgilin or deprenyl, according to the method described by Johnston et al. (1968 Biochem Pharm. 17:1285-1297).

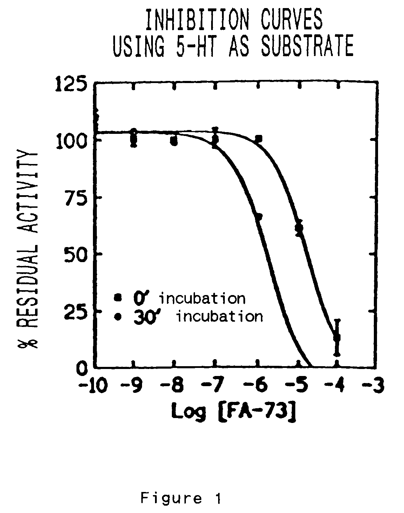

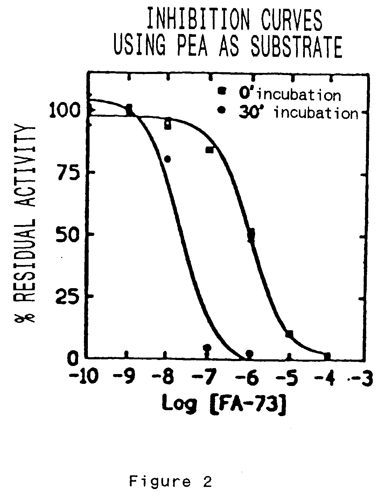

Determinations were made of the comparative MAO-A and MAO-B activity inhibition curves of the various compounds under study at time 0 and 30 min. by measuring the remaining activity to 5-HT and PEA as substrates. The graphs corresponding to the compound FA-73 (see Fig 1 and 2) have been taken as representative of the whole series of 5-benzyloxy derivatives.

The IC₅0 values corresponding to 5-benzyloxy derivatives are shown in Table 1.

These values are within the nano-molar and micro-molar range and also show that compounds of 5-benzyloxy derivatives are selective of the MAO-B form.

The comparative study of the inhibition curves of these compounds shows, in all cases, an inhibition mechanism that is dependent on the incubation time, as well as a greater selectivity for the MAO-B form.

### Example 2. Confirmation of the time dependence of the inhibition process in experimental animals.

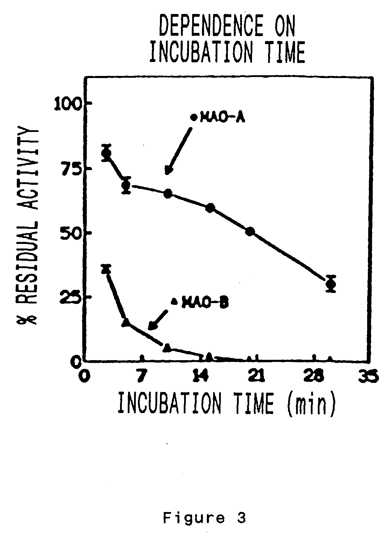

Figure 3 shows the dependence of the process on the incubation time for compound FA-73. This compound has been selected as representative of all the inhibitors studied as they showed similar behaviour. The graph clearly shows the drop in the formation of product as a function of the time of incubation with the inhibitor for both enzymatic forms.

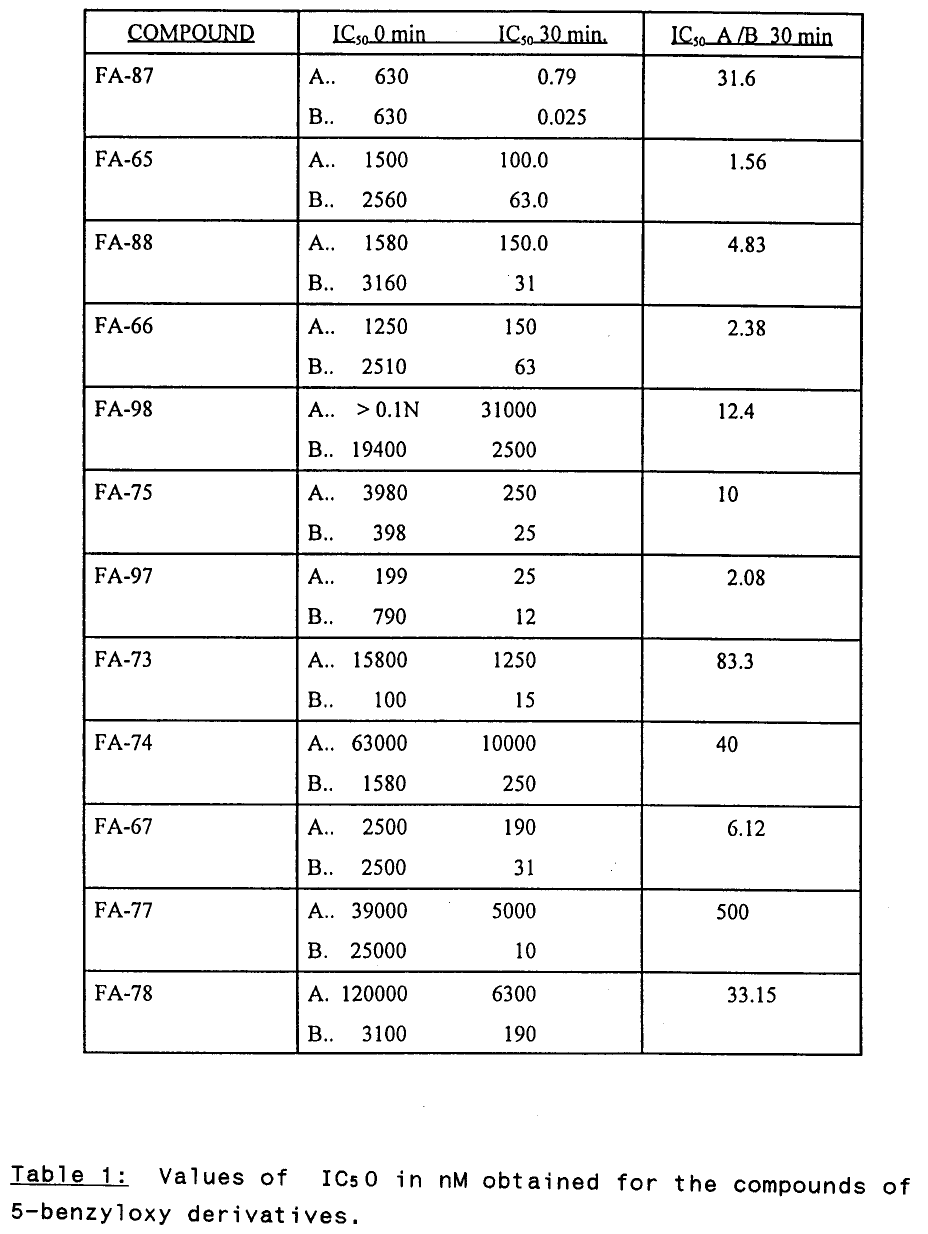

### Example 3. Determination of the irreversibility of the inhibition process in experimental animals.

The studies of the irreversibility of the inhibition of MAO-A and MAO-B activity were carried out in each case using concentrations close to the IC₅0 of each compound. This means that, at the selected inhibitor concentration range, any recuperation of enzymatic activity, if it occurs and in the event that the inhibition process is reversible, is easily observed.

The remaining MAO-A and MAO-B activity did not increase appreciably in any of the compounds studied, indicating the irreversibility of the inhibition process.

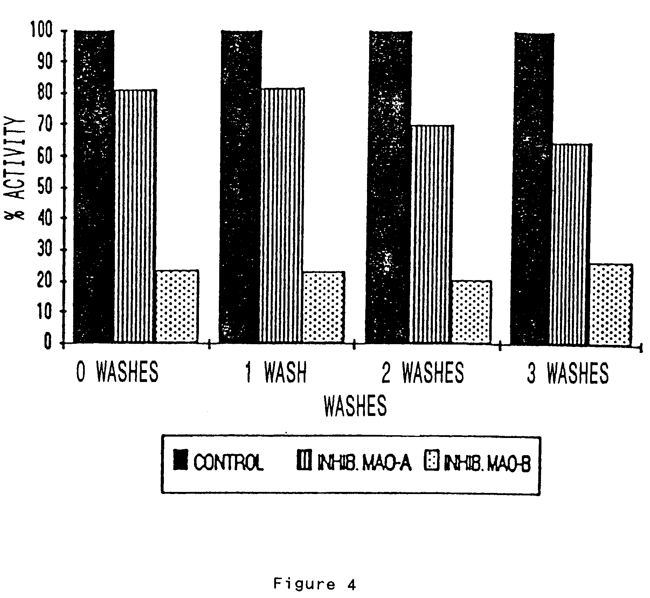

Figure 4, showing the reversibility study made using the compound FA-73, is representative of the whole series of compounds, indicating irreversibility in all the cases studied.

### Example 4. Determination of the kinetic parameters in experimental animals.

The results mentioned above indicate that these compounds behave as suicide type inhibitors for both forms of MAO, according to the following mechanism: 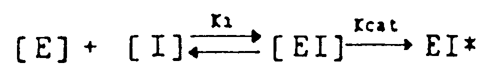 These compounds have a structure analogous to that of the substrate and are recognised as such by the active core of the enzyme being transformed into a reactive species that generates a covalent irreversible complex.

Based on the apparent velocity constants of the first order (kapp) obtained for each inhibitor, calculations were made of the K₁ and K¹_{c} at (according to the method described by M. Avila et al. 1993, Biochem Pharmac 45, 2231-2237). K₁ is the affinity constant of the first stage of the reaction and K₁ MAO-A / K₁ MAO-B the expression of the selectivity.

K¹_{c}at is the velocity constant for the formation of the covalent irreversible complex during the second stage of the process. To compare the values of these constants towards the two forms of MAO, we express the inhibition efficacy as the ratio K¹_{c}at/K₁. The value of these constants (see Tables 2 and 3), is the result of three different experiments, with their corresponding SEM.

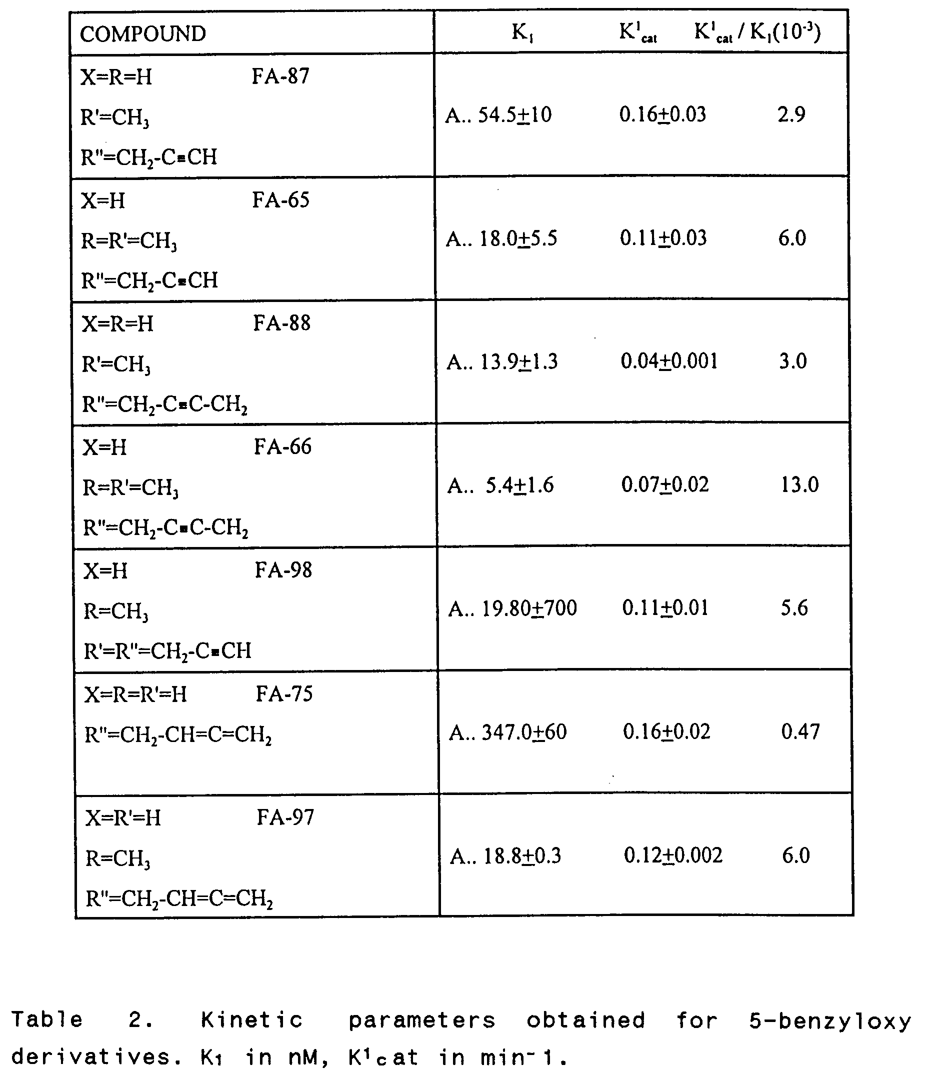

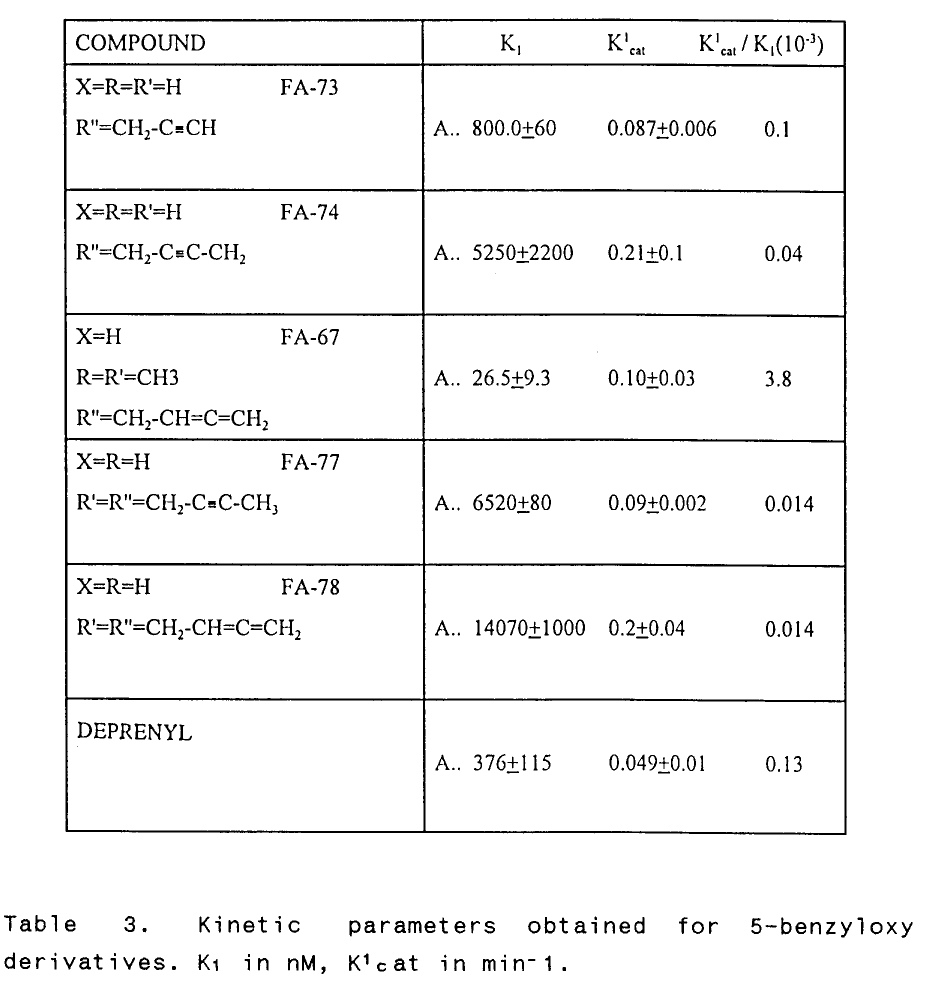

The results obtained in these tables leads to the conclusion that several of these compunds have a greater selectivity for MAO-B compared to deprenyl:

| | |
|---|---|
| DEPRENIL ... K₁ MAO-A/K₁ MAO-B = | 23.5 |
| FA-98 ........................ = | 873.5 |
| FA-75 ........................ = | 63.0 |
| FA-73 ........................ = | 1066.6 |
| FA-74 ........................ = | 276.4 |
| FA-77 ........................ = | 41.5 |
| FA-78 ........................ = | 913.6 |
| FA-67 ........................ = | 22.4 |

The catalytic efficacy values shown in the tables are, in the majority of cases, greatly superior to those obtained for deprenyl, the most significant being FA-73.

### Example 5. Re-uptake kinetic studies in experimental animals.

Kinetic studies of dopamine re-uptake were carried out in purified synaptosomes of brain striatal tissue from Sprague-Dawley rats. The compound FA-73 was used as being representative of all the 5-benzyloxy derivatives studied and compared to deprenyl, a highly MAO-B specific agent used in Parkinsonism therapy.

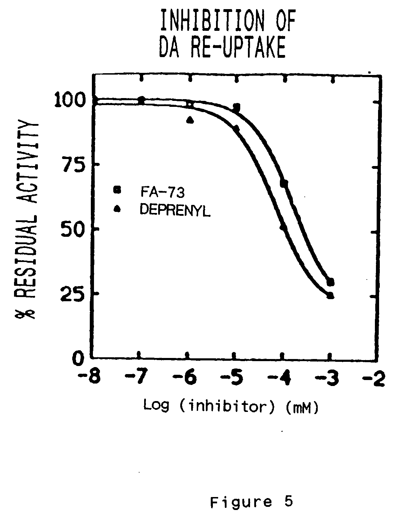

Figure 5 shows the remnant activity of the dopamine uptake carrier when incubated in the presence of the inhibitors FA-73 and deprenyl. Considering the kinetic constants obtained, the substrate concentration was 0.1 µM.

The values of IC₅0 (mM) obtained are the following:

| COMPOUND | FA-73 | DEPRENYL |
|---|---|---|
| IC₅₀ | 0.15 | 0.068 |

These results indicate that the compound FA-73 presents an inhibition sensitivity to the dopamine carrier in rat striate of the same order as that shown by deprenyl.

### Example 6. Determination of the IC₅0 on MAO-A and MAO-B activity in humans.

The determination of the IC₅0 was performed using homogenated human cerebral cortex obtained from autopsies carried out within the 10 hours following the death of persons aged between 35 and 55 years. They were provided by Dr. Fco Javier González Oliván (Forensic Anatomic Institute of the Hospital Clínico Provincial in Barcelona).

The values obtained at time 0 minutes of incubation with the 5-benzyloxy derivative FA-73 were as follows:
IC₅0 MAO-A20,000 nMIC₅0 MAO-B200 nM

These values indicate that the selective inhibition of these compounds for the MAO-B form is maintained in human tissue (100 fold more selective) with values of IC₅0 in the same order as those obtained at time 0 in liver of Sprague-Dawley rat.

### Example 7. Determination of the IC₅0 of the dopamine carrier in humans.

This assay was carried out in purified synaptosomes of human caudate obtained using the same method as described by Rodríguez et al. 1987 Biochem Pharmac 36, 974-976.

The studies were carried out with the same inhibitors used in the rat tissue. The dopamine (DA) concentration was 0.1 µM, a value chosen from the kinetic constants obtained.

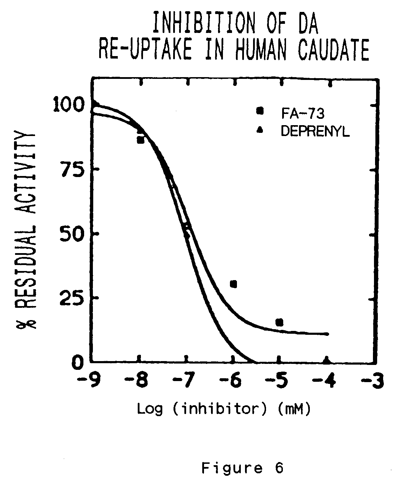

The values of IC₅0 shown below were obtained from the resulting inhibition curves (see Fig. 6).

| COMPOUND | FA-73 | DEPRENYL |
|---|---|---|
| IC₅₀₍ₘₐₓ₎ | 0.36 | 0.1 |

These values, obtained for the sensitivity towards the dopamine carrier in human caudate, indicate that the 5-benzyloxy derivative used behaves in a way similar to deprenyl and has similar values for the IC₅0.

These values of IC₅0 indicate the enormous sensitivity of these compounds in this tissue, with a inhibition power 1,000 times greater than that shown in rat striate.

### Example 8. "Ex Vivo" pharmacological assays of selective inhibition of MAO-B in experimental animals.

The pharmacological assays were carried out on C57/BL mice. These animals are sensitive to the induction of the symptomatology of Parkinson's disease through the action of MPTP. The animals were distributed into three groups: one group was used as a control; another group was injected with l-deprenyl (10 mg/Kg) by intraperitoneal route and the third group was administered the compound being tested (4 mg/Kg).

The animals were sacrificed by decapitation after 2 hours and the brains homogenised in potassium phosphate buffer 50 mM, pH 7.2. Determinations were made of the MAO-A and MAO-B activity in each case using radiometric methods.

The results with compound FA-73 were the following:

| | MAO-B ACTIVITY | MAO-A ACTIVITY |
|---|---|---|
| CONTROL | 303 (100%) | 485 (100%) |
| DEPRENYL | 15 (4.8%) | 255 (52.5%) |
| FA-73 | 16 (5.3%) | 467 (96.2%) |

The results indicate that:
- Compound FA-73 crosses the hematoencephalic barrier.
- In the brain of C57/BL mouse the selectivity of MAO-B inhibition shown in Sprague-Dawley rat and human cerebral cortex is maintained.
- At the doses used, it is noted that compound FA-73 inhibits MAO-B activity in the same order as deprenyl. However, MAO-A activity is practically unaffected by this compound whereas it is reduced to almost 50% when deprenyl is administered.

The other compounds assayed showed similar characteristics.

### Example 9. Protection against the neurotoxicity of MPTP in experimental animals.

One group of 3 mice of the C57/BL species, sensitive to MPTP neurotoxin that produces the symptoms of Parkinsonism, was treated with compound FA-73 by intraperitoneal injection (3 mg/Kg). Two hours later they were administered MPTP HCl (30 mg/Kg i.p.) and this dose was repeated on the second day. Twenty four hours after the last injection of MPTP the animals were sacrificed. The brains were dissected and determinations made of the dopamine (DA) and 3,4-dihydroxyphenylacetic acid (DOPAC) levels in the striate. No significant differences were observed between treated and non-treated animals:

| | Dopamine (DA) µg/g dry tissue | DOPAC µg/g dry tissue |
|---|---|---|
| Control animals | 10.85 ± 0.1 | 0.98 ± 0.03 |
| Animals with FA-73+MPTP | 10.48 ± 0.085 | 0.93 ± 0.023 |
| Animals treated with MPTP | 3.13 ± 0.018 | 0.54 ± 0.005 |

### Example 10. Protection against the neurotoxicity of Ibotenic acid.

One group of Sprague-Dawley rats was treated with compound FA-73 (3 mg/Kg, i.p.) followed two hours later by an stereotaxic injection of ibotenic acid into the cerebral ventricles at a dose that would induce an Alzheimer type of pathology. After sacrificing the animals, determinations were made of the acetylcholine (AC) in the homogenised brains.

At the same time, a second group of animals was only treated with ibotenic acid and a third group did not receive any treatment and was used as a control.

The results showed that, taking the acetylcholine levels of the control animals as 100%, this level in the animals treated with ibotenic acid was 3% and that of the animals treated with compound FA-73 and ibotenic acid up to 85% of the control, depending on the dose administered.

Similar results were obtained with some of the other compounds.

### Example 11. Forms of administration.

1. For treatment of Parkinsonism, in tablets by oral route:

| | |
|---|---|
| L-Dopa (Levadopa) | 250 mg |
| Benserazide (or carbidopa) | 50 mg |
| FA-73 | 3 mg |
| Lactose and other excipients, up to 100 mg. | |

2. As general neuroprotectors in tablets by oral route:

| | |
|---|---|
| FA-73 | 5 mg |
| Lactose and other excipients, up to 100 mg. | |

### Example 12. Formula of the 5-benzyloxy derivatives, derivative FA-73 in particular.

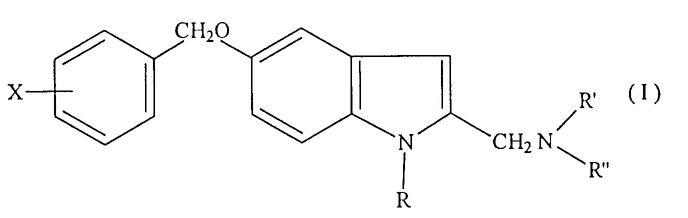

- FA-73: X = H
R = H
R' = CH₂-C-CH
R'' = H

### LEGEND

Figure 1: Inhibition sensitivity of compound FA-73 using 5-HT as substrate. All the 5 benzyloxy derivatives considered show the same behaviour.
Figure 2: Inhibition sensitivity of compound FA-73 using PEA as substrate. All the 5 benzyloxy derivatives considered show the same behaviour.
Figure 3: Dependency of the inhibition on the incubation time (FA-73).
Figure 4: Representation of the irreversibility of the inhibition process, FA-73 being chosen as an example for all the derivatives studied.
Figure 5: Inhibition curves using deprenyl and FA-73 as inhibiting agents.
Figure 6: Inhibition curves. Activity of the DA carrier in % compared to increasing inhibitor concentrations.

## Claims

1. Pharmaceutical compositions based on aminomethylindoles for therapeutic application as neuroprotectors in Parkinson's and Alzheimer's disease containing, as an active ingredient, a compound derived from 2-aminomethyl-5-benzyloxyindoles represented by the general formula (I): 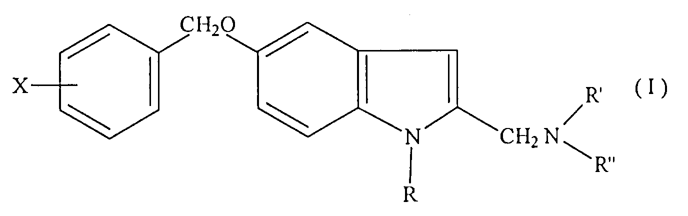 where X may be located at any of the positions of the benzene ring and may be hydrogen (H), a halogen (F, Cl, Br, l), an alkoxy group such as CH₃O, an alkylthio group such as CH₃S, an alkyl group such as CH₃, an aryl group such as C₆H₅, which may be substituted or not, an aryloxy group such as C₆H₅₀, an arylthio group such as C₆H_{5S}, an amino, alkylamino or dialkylamino group such as NH₂, CH₃HN, (CH₃)₂N, a hydroxyl group (OH), or any possible combination thereof; R may be hydrogen (H), methyl (CH₃) or another C₁₋₃ aliphatic or alicyclic or aromatic radical such as C₆H₅, which may be substituted or not, an arylalkyl group; R' may be hydrogen (H), methyl (CH₃) or another C₁₋₃ aliphatic or alicyclic radical or an aryl or arylalkyl radical or a radical similar to those indicated for R''; R'' may be hydrogen (H), methyl (CH₃) or a C₁₋₃ aliphatic or alicyclic radical or an acetylene or alene group including those represented by the propinyl radicals (CH=C-CH₂), 2-butinyl (CH₃-C=C-CH₂), or 2,3-buta-dienyl (CH₂=C=CH-CH₂) including any of their salts or pharmaceutically acceptable complexes, all of them being potent selective monoamine oxidase B inhibitors.

2. The pharmaceutical compositions of claim 1, with a possible neuroprotective effect in Parkinson's disease, since they show a high inhibition potency and selectivity for MAO-B, further to their inhibitory action on the dopaminergic re-uptake system.

3. The pharmaceutical compositions of claim 1, which, being MAO-B inhibitors inside these terminals through the dopamine carrier, show a possible rivalry with the MPP⁺ neurotoxin, impeding its entry and preventing its damaging effect that would lead to the degeneration of the dopaminergic terminals.

4. The pharmaceutical compositions of claim 1 which, when administered by intraperitoneal route, easily penetrate the brain of C57/BL mice, selectively inhibiting MAO-B and, therefore preventing the formation of the neurotoxic metabolites from MPTP and impeding the dopaminergic degeneration characteristic of Parkinson's disease.

5. Pharmaceutical compositions based on aminomethylindoles containing any of the agents recited in claim 1 as an active ingredient, in any form used pharmaceutically such as solutions, tablets, suspensions or ointments and in any type of composition, either singly or in association with other biologically active compounds including pharmaceutically acceptable excipients, to be used as neuroprotective agents.

6. The pharmaceutical compositions based on aminomethylindoles of claims 1 and 2, in any of their forms and singly or associated with other compounds and excipients to be used in the treatment of neurodegenerative processes and especially those known as Parkinson's and Alzheimer's disease.
